# EUROPEAN PATENT APPLICATION

(11) **EP 1 586 267 A1**
(43) Date of publication of application: **19.10.2005**
(21) Application number: 05007409.5
(22) Date of filing: 05.04.2005
(51) Int. Cl.: A61B 5/053

(54) **Body composition measuring apparatus**

(30) Priority: 13.04.2004 JP 2004118269
(71) Applicant: TANITA CORPORATION, Tokyo (JP)
(72) Inventor: Koyama, Kazuyasu, Itabashi-ku Tokyo (JP)
(74) Representative: Grünecker, Kinkeldey, Stockmair & Schwanhäusser Anwaltssozietät

(57) **Abstract**

A body composition measuring apparatus is a body composition measuring apparatus which comprises : at least a pair of current passing electrodes for passing a current between two body parts of a user, at least a pair of voltage measuring electrodes for measuring a potential difference between the two body parts, and a main unit having a surface on which these electrodes are disposed such that they are insulated from each other, wherein the electrodes are disposed on the surface of the main unit such that each of the current passing electrodes makes contact with a body at multiple points sandwiching a contact point between the voltage measuring electrode and the body and each of the voltage measuring electrodes makes contact with the body at multiple points sandwiching a contact point between the current passing electrode and the body.
Fig. 2
5: DISPLAY
6: INPUT DEVICE
9: CURRENT SUPPLYING CIRCUIT
10: VOLTAGE MEASURING CIRCUIT
11: WEIGHT SENSOR
12: A/D CONVERTER
13: INPUT/OUTPUT CIRCUIT
14: STORAGE UNIT
15: POWER UNIT
16: CONTROL UNIT

## Description

### BACKGROUND OF THE INVENTION

### (i) Field of the Invention

The present invention relates to a body composition measuring apparatus for measuring a body composition of a user such as body fat, visceral fat, body water, a muscle, a bone orabasalmetabolism. More specifically, the present invention relates to a body compositionmeasuring apparatus which comprises at least a pair of current passing electrodes for passing a current between two body parts of a user, at least a pair of voltage measuring electrodes for measuring a potential difference between the two body parts, and a main unit having a surface on which these electrodes are disposed such that they are insulated from each other.

### (ii) Description of the Related Art

Owing to increasing awareness of healthcare in recent years, body composition measuring apparatuses for measuring a body composition (hereinafter generically and simply referred to as "body composition") of a user such as body fat, visceral fat, body water, a muscle, a bone or a basal metabolism have been developed and actually used. In particular, a body composition measuring apparatus comprising at least a pair of current passing electrodes for passing a current between two body parts of a user, at least a pair of voltage measuring electrodes for measuring a potential difference between the two body parts and amainunit having a surface on which these electrodes are disposed such that they are insulated from each other (refer to Patent Publications 1, 2, 3 and 4, for example) measures a body composition based on the bioelectrical impedance of the user and is very popular in the market because of its supported ease of use and accuracy.
Patent Publication 1
Japanese Utility Model Publication No. 2514259
Patent Publication 2
Japanese Patent Laid-Open Publication No. 2001-78978
Patent Publication 3
Japanese Patent Laid-Open Publication No. 2001-228013 Patent Publication 4
U.S. Patent No. 6473643

In the above conventional body composition measuring apparatus, the current passing electrodes and the voltage measuring electrodes make contact with body parts such as the bottoms of the feet or palms inpairs, and when the contactposition is displaced, measurement results vary significantly. The variation in measurement is assumed to be attributed to a variation in the contact area between the body part and the current passing electrode and/or the measuring electrode. That is, for example, in the apparatus described in Patent Publication 1, when a user stands on a platform closer to the front side of the platform (i.e., stands with the bottoms of the feet covering smaller portions of the electrodes for the heel side than when the user stands at the normal position), the contact areas between the bottoms of the feet and the electrodes for the toe side are relatively larger than those between the bottoms of the feet and the electrodes for the heel side, as compared with when the user stands closer to the back side of the platform (i.e., stands with the bottoms of the feet covering smaller portions of the electrodes for the toe side than when the user stands at the normal position). It is assumed that the variation in the contact area affects bioelectrical impedances including a contact impedance between a body part and an electrode, thereby causing a variation in the final results of measurements of body compositions.

Hence, to suppress the variation in the measurements, guides, pits, projections or the like for controlling contact positions between electrodes and the bottoms of the feet and/or the palms are formed around or on the electrodes in the apparatuses described in Patent Publications 1 to 3. However, despite formation of the guides, pits or projections, it is cumbersome for users to care contact positions between the bottoms of the feet or the palms and the electrodes in using the apparatuses. Further, in the case of an apparatus such as the apparatus described in Patent Publication 4 which does not even have the guides, pits or projections formed thereon and contains only a simple note of caution about contact positions between the bottoms of the feet and electrodes in its instruction manual or the like, a user must check the accuracy of contact positions onhis/herownforeachmeasurement. Inotherwords, the greatest advantage of apparatuses using a bioelectrical impedance, i.e., ease of use, is significantly impaired, whereby the feeling of a user who intends to use a body composition measuring apparatus casually for better healthcare may be blighted.

Such cumbersomeness as described above is particularly strong in the case of an apparatus which contacts electrodes with the bottoms of the feet. The reason is that in the case of the apparatus which contacts electrodes with the bottoms of the feet, a user must check the positions of his feet, standing on the top surface of the apparatus, and it is therefore difficult to check the contact condition of the feet with the electrodes, as compared with an apparatus which allows a user to contact his palms with electrodes before his eyes.

Further, if the above variation in the measurements is caused by the variation in the contact areas between the body parts and the electrodes, measurement results still vary depending on the size of body parts which make contact with the electrodes, even if the contact positions between body parts and the electrodes are controlled by such guides, pits or projections as disclosed in Patent Publications 1 to 3. More specifically, for example, in the case of the apparatus described in Patent Publication 1, when a user A with a large foot size and a user B with a small foot size make a measurement at an appropriate standing position by use of heel guides, the contact areas between the electrodes for the heel side and the bottoms of the feet of the users A and B are not so different while the contact areas between the electrodes for the toe side and the bottoms of the feet of the user A are larger than those of the user B, and this may affect the final results of measurements of body compositions and cause a decrease in measurement accuracy for either of the users.

Furthermore, the guides, pits andproj ections as disclosed in Patent Publications 1 to 3 have another problem of impairing the design of the whole apparatus including the electrodes and the flexibility of the design. For instance, when transparent electrode films are disposed on a transparent platform as in the apparatus described in Patent Publication 3, the design of the whole apparatus may be impaired if projections for determining the standing position are formed on the electrodes.

An object of the present invention is to provide a body composition measuring apparatus which can solve various problems as described above assumed to be caused by variations in contact position and contact area between body parts and electrodes, suppress a variation in measurement, make users feel no cumbersomeness and allow a high degree of flexibility in design.

### SUMMARY OF THE INVENTION

A body composition measuring apparatus of the present invention comprises:
at least a pair of current passing electrodes for passing a current between two body parts of a user,
at least a pair of voltage measuring electrodes for measuring a potential difference between the two body parts, and
a main unit having a surface on which these electrodes are disposed such that they are insulated from each other,
wherein
the electrodes are disposed on the surface of the main unit such that each of the current passing electrodes makes contact with a body at multiple points sandwiching a contact point between the measuring electrode and the body and each of the voltage measuring electrodes makes contact with the body at multiple points sandwiching a contact point between the current passing electrode and the body.

The above current passing electrodes and the above voltage measuring electrodes may be disposed on the top surface of the main unit in pairs so that they make contact with the bottoms of the left and right feet of the user to pass a current between both feet and measure a potential difference between both feet.

Further, the above current passing electrodes and voltage measuring electrodes which make contact with the bottoms of the feet of the user in pairs each may be formed in the form of a comb when viewed from above and may be disposed such that their teeth engage each other.

Alternatively, the above current passing electrodes and voltage measuring electrodes which make contact with the bottoms of the feet of the user in pairs each may be formed in the form of small pieces when viewed from above and may be disposed such that small electrode pieces for passing a current and small electrode pieces for measuring a voltage are disposed alternately.

Further, the above small electrode pieces may be round or stick-shaped when viewed from above.

In the body composition measuring apparatus of the present invention, the current passing electrodes and the voltage measuring electrodes are disposed on the surface of the main unit such that they engage the opposed electrodes. With the thus disposed electrodes, the total contact area between the electrodes and a body does not vary significantly even if the contact positions between the electrodes and body parts are somewhat displaced. Thus, the body composition measuring apparatus of the present invention can reduce a variation in contact areas between the electrodes and body parts regardless of contact positions between the electrodes and body parts and suppress a variation in measurements caused by the variation in the contact areas. As a result, the contact positions between the electrodes and body parts need not be strictly controlled, so that users feel no cumbersomeness and guides, pits or projections for controlling the contact positions can be eliminated to improve the flexibility of the design of the apparatus.

Further, in the body composition measuring apparatus of the present invention, the above current passing electrodes and the above voltage measuring electrodes can be disposed on the top surface of the above main unit in pairs so that they make contact with the bottoms of the left and right feet of a user to pass a current between both feet and measure a potential difference between both feet. With the above configuration of the electrodes, cumbersomeness in using a conventional body composition measuring apparatus of a type which contacts the bottoms of the feet with electrodes and gives particularly strong cumbersomeness to users can be eliminated.

Further, the above current passing electrodes and the above measuring electrodes can adopt various designs. For instance, they each may be formed in the form of a comb when viewed from above and be disposed such that their teeth engage each other, or they each may be formed in the form of small round or stick-shaped pieces when viewed from above and be disposed such that those small electrode pieces for passing a current and small electrode pieces for measuring a voltage are disposed alternately.

### BRIEF DESCRIPTION OF THE DRAWINGS

Fig. 1 is a schematic external view of a body composition measuring apparatus 1 according to the present invention.
Fig. 2 is a block diagram showing a general description of the configuration of an electric circuit incorporated in the body composition measuring apparatus 1.
Fig. 3 is a diagram for illustrating a position where a user stands in using the body composition measuring apparatus 1.
Fig. 4 is a diagram for illustrating various embodiments of the present invention.
Fig. 5 is another diagram for illustrating various embodiments of the present invention.
Fig. 6 is a schematic external view of a conventional body composition measuring apparatus 1' used in a comparative test for confirming the effect of the present invention.

### DETAILED DESCRIPTION OF THE PREFERRED EMBODIMENTS

A body composition measuring apparatus of the present invention comprises:
at least a pair of current passing electrodes for passing a current between two body parts of a user,
at least a pair of voltage measuring electrodes for measuring a potential difference between the two body parts, and
a main unit having a surface on which these electrodes are disposed such that they are insulated from each other,
wherein
the electrodes are disposed on the surface of the main unit such that each of the current passing electrodes makes contact with a body at multiple points sandwiching a contact point between the voltage measuring electrode and the body and each of the voltage measuring electrodes makes contact with the body at multiple points sandwiching a contact point between the current passing electrode and the body.

The above current passing electrodes and the above voltage measuring electrodes may be disposed on the top surface of the main unit in pairs so that they make contact with the bottoms of the left and right feet of the user to pass a current between both feet and measure a potential difference between both feet.

Further, the above current passing electrodes and voltage measuring electrodes which make contact with the bottoms of the feet of the user in pairs each may be formed in the form of a comb when viewed from above and may be disposed such that their teeth engage each other.

Alternatively, the above current passing electrodes and voltage measuring electrodes which make contact with the bottoms of the feet of the user in pairs each may be formed in the form of small pieces when viewed from above and may be disposed such that small electrode pieces for passing a current and small electrode pieces for measuring a voltage are disposed alternately.

Further, the above small electrode pieces may be round or stick-shaped when viewed from above.

### Embodiments

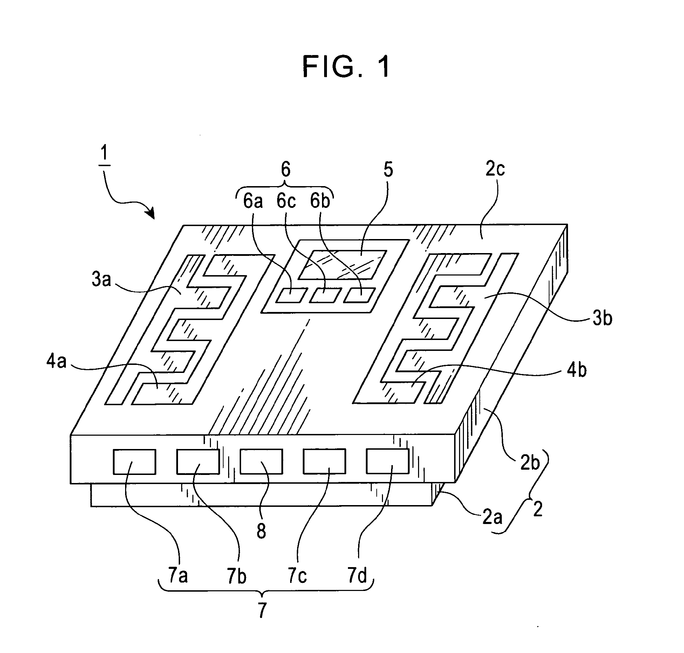

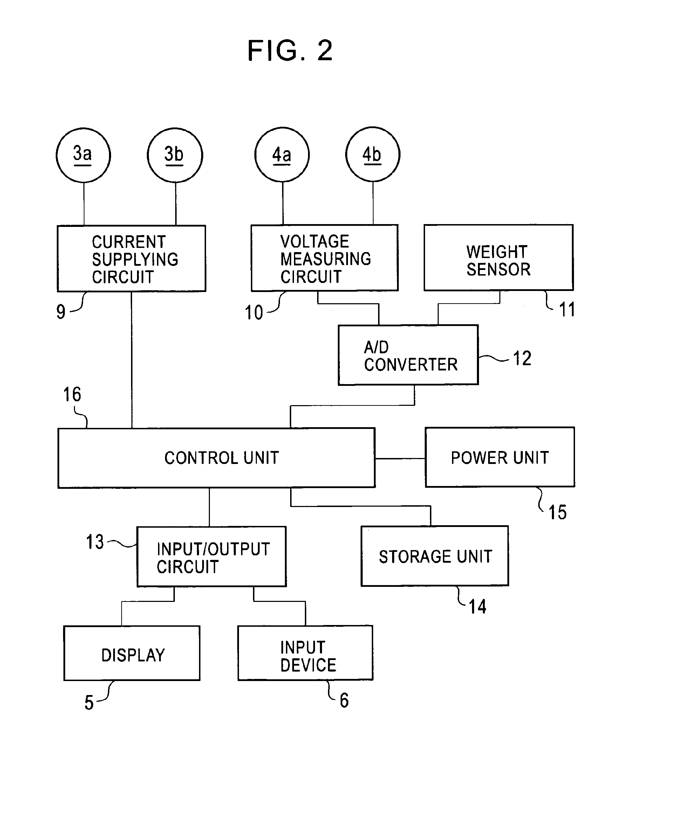

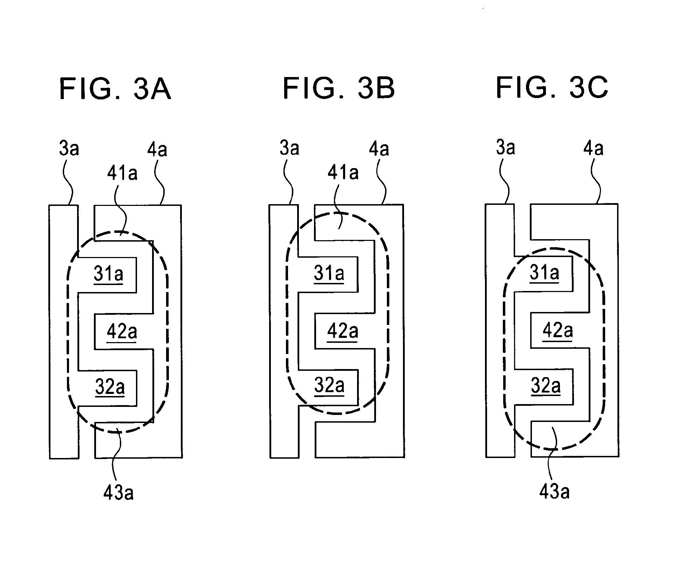

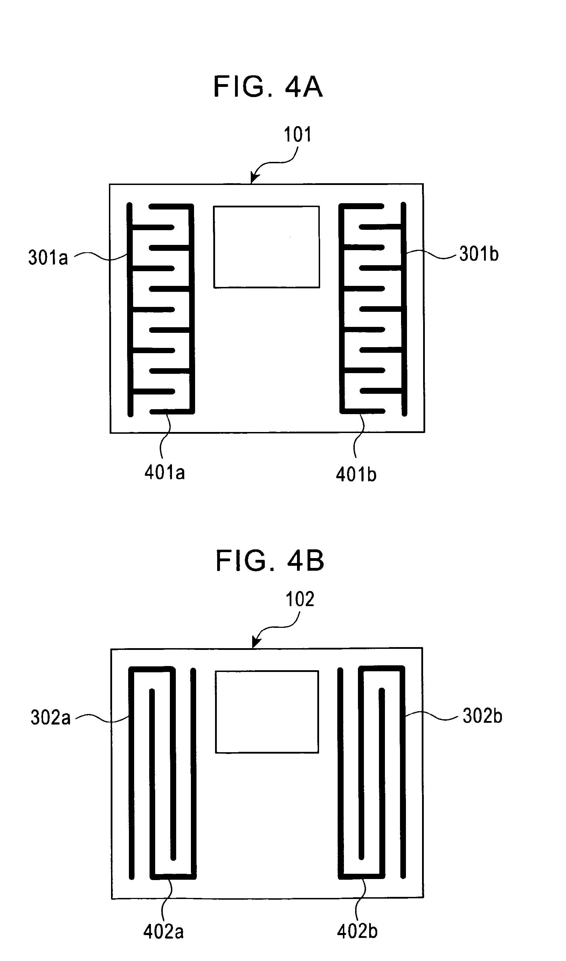

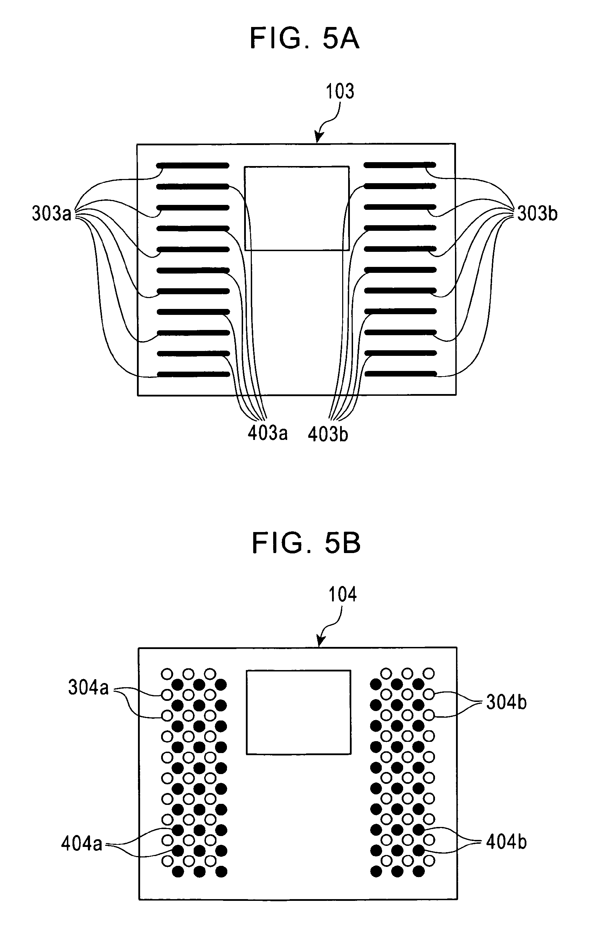

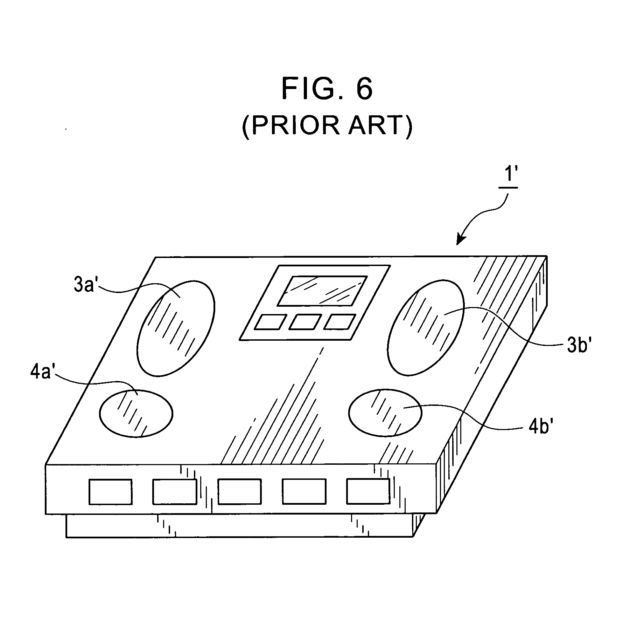

Hereinafter, a suitable embodiment of the present invention will be described with reference to the drawings. Fig. 1 is a schematic external view of a body composition measuring apparatus 1 according to the present invention. Fig. 2 is a block diagram showing a general description of the configuration of an electric circuit incorporated in the body composition measuring apparatus 1. Fig. 3 is a diagram for illustrating a position where a user stands in using the body composition measuring apparatus 1. Figs. 4 and 5 are diagrams for illustrating various embodiments of the present invention. Fig. 6 is a schematic external view of a conventional body composition measuring apparatus 1' used in a comparative test for confirming the effect of the present invention.

As shown in Fig. 1, the body composition measuring apparatus 1 comprises a main unit 2 which comprises a base 2a and a platform 2b. The platform 2b is mounted on the base 2a. On the top surface 2c of the platform 2b, there are provided current passing electrodes 3a and 3b for passing an alternating current between the bottoms of the left and right feet as two body parts of a user, voltage measuring electrodes 4a and 4b formeasuringavoltage (potential difference) occurring between the bottoms of the left and right feet due to the current passing, a display 5 which is used to, for example, display a body composition measured by the body composition measuring apparatus 1 and comprises a known liquid crystal screen, and an input device 6 which is used by the user to enter personal data such as gender, age and a body height. The top surface 2c of the platform 2b is made of resin, and the current passing electrodes 3a and 3b and the voltage measuring electrodes 4a and 4b are insulated from each other on the top surface 2c. The input device 6 comprises selection keys 6a and 6b which are used to, for example, select data to be input, and a setting key 6c which is used to, for example, enter data to be input. Further, on a side face of the main unit 2, personal keys 7 which comprise four keys 7a, 7b, 7c and 7d for retrieving already entered personal data and a power key 8 are provided.

Further, as shown in Fig. 2, in the main unit 2 of the body composition measuring apparatus 1, a current supplying circuit 9 which is connected to the above current passing electrodes 3a and 3b, a voltage measuring circuit 10 which is connected to the above voltage measuring electrodes 4a and 4b, a weight sensor 11 which measures the body weight of the user by outputting a voltage corresponding to a load, an A/D converter 12 which digitizes voltage signals from the voltage measuring circuit 10 and the weight sensor 11, an input/output circuit 13 which is connected to the above display 5 and input device. 6, a storage unit 14 which stores enteredpersonal data, a measured body weight and measured body compositions, a power unit 15 which comprises batteries, and a control unit 16 which is electrically connected to these current supplying circuit 9, A/D converter 12, input/output circuit 13, storage unit 14 and power unit 15 are provided.

In such a constitution, the control unit 16 comprises a known computing device (CPU) and executes control programs stored in advance in the storage unit 14 to perform various control processes such as control of supplying an alternating current to the current passing electrodes 3a and 3b, control of calculating the bioelectrical impedance of the user based on this current value and a voltage value detected by the voltage measuring electrodes 4a and 4b, control of calculating the body composition of the user based on this bioelectrical impedance, the user's personal data entered by means of the input device 6 and the user's body weight measured by the weight sensor 11, and control of displaying the entered personal data, the measured body weight and the calculated body composition on the display 5 and storing these data in the storage unit 14, thereby measuring the body composition of the user. Further descriptions of these control processes will be omitted since they are the same as those for a conventionally known body composition measuring apparatus.

A characteristic constitution of the body composition measuring apparatus 1 which is an embodiment of the present invention consists in the configuration of its electrodes. First, the current passing electrode 3a and the voltage measuring electrode 4a are disposed, as a pair, on the top surface 2c of the main unit 2 so that they make contact with the bottom of the left foot of the user. Further, these electrodes 3a and 4a each are formed in the form of a comb when viewed from above and are disposed such that their teeth 31a, 32a, 41a, 42a and 43a (refer to Fig. 3) engage each other. Similarly, the current passing electrode 3b and the voltage measuring electrode 4b are disposed, as a pair, on the top surface 2c of the main unit 2 so that they make contact with the bottom of the right foot of the user, and these electrodes 3b and 4b each are formed in the form of a comb when viewed from above and are disposed such that their teeth engage each other.

The user stands on the top surface 2c of the main unit 2 with the bottoms of the left and right feet in contact with the electrodes 3a, 4a, 3b and 4b disposed as described above to measure a body composition. The position where the user stands at that time may differ as shown in Fig. 3 for each measurement. In Fig. 3, ellipses drawn by dotted lines schematically show areas where the left foot of the user is placed. The electrodes 3a and 4a each make contact with the bottom of the left foot of the user inside this oval. That is, Fig. 3(a) shows that the left foot of the user is placed nearly in the center of the pair of electrodes, Fig. 3(b) shows that the standing position is off the oval shown in Fig. 3(a) in the toe direction, and Fig. 3(c) shows that the standing position is off the oval shown in Fig. 3(a) in the heel direction.

In any of the cases of Figs. 3(a) to 3(c), the current passing electrode 3a makes contact with the bottom of the foot by the tooth 31a and the tooth 32a which are disposed with the tooth 42a sandwiched therebetween. The tooth 42a includes a contact point between the voltage measuring electrode 4a and the bottom of the foot. That is, it can be said that the contact area between the current passing electrode 3a and the bottom of the foot is nearly the same in any of the cases of Figs. 3(a) to 3(c) .

Meanwhile, the voltage measuring electrode 4a makes contact with the bottom of the foot, by a portion of the tooth 41a, the tooth 42a and a portion of the tooth 43a in the case of Fig. 3(a), by a large portion of the tooth 41a and the tooth 42a in the case of Fig. 3(b), and by the tooth 42a and a large portion of the tooth 43a in the case of Fig. 3(c): That is, it can be said that the contact area between the voltage measuring electrode 4a and the bottom of the foot is also nearly the same in any of the cases of Figs. 3(a) to 3(c). The tooth 41a and the tooth 42a are disposed with the tooth 31a including a contact point between the current passing electrode 3a and the bottom of the foot, sandwiched therebetween, and the tooth 42a and the tooth 43a are disposed with the tooth 32a including a contact point between the current passing electrode 3a and the bottom of the foot, sandwiched therebetween.

The above described configuration of the current passing electrode 3a and the voltage measuring electrode 4a is also applied to the current passing electrode 3b and the voltage measuring electrode 4b which make contact with the bottom of the right foot.

As described above, this body composition measuring apparatus 1 comprises the current passing electrodes 3a and 3b for passing a current between the bottoms of both feet as two body parts, the voltage measuring electrodes 4a and 4b for measuring a potential difference between both feet, and the main unit 2 which has the top surface 2c on which these electrodes 3a, 3b, 4a and 4b are insulated from each other. The electrodes 3a, 3b, 4a and 4b are disposed on the top surface 2c of the main unit 2 such that the above current passing electrodes 3a and 3b each make contact with the bottom of the foot at multiple points (such as a point included in the tooth 31a and a point included in the tooth 32a) which sandwich a contact point (such as a point included in the tooth 42a) between the voltage measuring electrode and the bottom of the foot, and the above voltage measuring electrodes 4a and 4b each make contact with the bottom of the foot at multiple points (such as a point included in the tooth 41a and a point included in the tooth 42a or a point included in the tooth 42a and a point included in the tooth 43a) which sandwich a contact point (such as a point included in the tooth 31a or a point included in the tooth 32a) between the current passing electrode and the bottom of the foot. Consequently, even when the contact position of the bottom of the foot is displaced from the position shown in Fig. 3(a) to the position shown in Fig. 3(b) or (c), the entire contact area between each electrode and the bottom of the foot does not change significantly, and a variation in the contact area is reduced.

Figs.4 and 5 schematically illustrate various embodiments of the present invention.

A body composition measuring apparatus 101 in Fig. 4(a) uses current passing electrodes 301a and 301b and voltage measuring electrodes 401a and 401b which are formed in the form of a comb when viewed from above, as in the case of the above body composition measuring apparatus 1. However, these electrodes of the apparatus 101 have more teeth than the electrodes of the body composition measuring apparatus 1. The increase in the number of teeth can cause more effective absorption of displacement of contact position to suppress a variation in contact area.

A body composition measuring apparatus 102 in Fig. 4(b) has current passing electrodes 302a and 302b and voltage measuring electrodes 402a and 402b which are formed in the form of a comb in the longitudinal direction of the feet. It is assumed that the standing position is often displaced in the longitudinal direction of the feet, in the case of a body composition measuring apparatus of a type which contacts electrodes with the bottoms of the feet. Accordingly, with the comb-shaped electrodes which have teeth extending in the longitudinal direction of the feet, displacement of contact position caused by displacement of standing position can be absorbed more effectively to suppress a variation in contact area.

A body composition measuring apparatus 103 in Fig. 5(a) has current passing electrodes 303a and 303b and voltage measuring electrodes 403a and 403b which are disposed on the top surface of the main unit as a plurality of small independent stick-shaped electrode pieces such that each piece of the electrodes 303a and 403a is disposed alternately and each piece of the electrodes 303b and 403b is also disposed alternately. As a matter of course, the small electrode pieces as current passing electrodes 303a and 303b are electrically connected to one another, respectively, in the main unit and are connected to a current supplying circuit as in the case of the current passing electrodes 3a and 3b in Fig. 2. Further, the small electrode pieces as voltage measuring electrodes 403a and 403b are also electrically connected to one another, respectively, in the main unit and are connected to a voltage measuring circuit as in the case of the voltage measuring electrodes 4a and 4b in Fig. 2.

A body composition measuring apparatus 104 in Fig. 5(b) has current passing electrodes 304a and 304b (shown as white hollow circles in the drawing) and voltage measuring electrodes 404a and 404b (shown as black solid circles in the drawing) which are disposed on the top surface of the main unit as a plurality of small independent round electrode pieces such that each piece of the electrodes 304a and 404a is disposed alternately and each piece of the electrodes 304b and 404b is also disposed alternately. As a matter of course, the small electrode pieces as current passing electrodes 304a and 304b are electrically connected to one another, respectively, in the main unit and are connected to a current supplying circuit as in the case of the current passing electrodes 3a and 3b in Fig. 2. Further, the small electrode pieces as voltage measuring electrodes 404a and 404b are also electrically connected to one another, respectively, in the main unit and are connected to a voltage measuring circuit as in the case of the voltage measuring electrodes 4a and 4b in Fig. 2. The white and black circles in the drawing are used for convenience's sake, and such use of different colors is not required.

When electrodes are constituted by small electrode pieces as in Figs. 5(a) and 5(b), displacement of contact position can be absorbed more effectively to suppress a variation in contact area.

To confirm the effect of the present invention, the present applicant conducted a comparative test about a variation in measurement of body fat percentage on three users A, B and C who differed in age, body height, body weight and foot size, by use of the body composition measuring apparatus 101 which has the comb-shaped electrodes 301a, 301b, 401a and 401b having a number of teeth as shown in Fig. 4(a) and a conventionally known body composition measuring apparatus 1' which has current passing electrodes 3a' and 3b' disposed on the toe side and measuring electrodes 4a' and 4b' disposed on the heel side as shown in Fig. 6.

In the comparative test, the users A, B and C used each body composition measuring apparatus to measure a body fat percentage at three dif ferent standing positions, i.e., at nearly the center, the front side and the rear side of the disposed electrodes as shown in Fig. 3. Of the three body fat percentages measured by the user A using the conventionally known body composition measuring apparatus 1', the maximum value was 22.4% and the minimum value was 21.4%, and the difference between the maximum and minimum values was 1 point. Meanwhile, the difference between the maximum and minimum values when the user A used the body composition measuring apparatus 101 according to the present invention was 0.1 points. Similarly, the difference between the maximum and minimum values when the user B used the conventionally known body composition measuring apparatus 1' was 0.7 points, and the difference between the maximum and minimum values when the user B used the body composition measuring apparatus 101 according to the present invention was 0.4 points. Similarly, the difference between the maximum and minimum values when the user C used the conventionally known body composition measuring apparatus 1' was 0. 7 points, and the difference between the maximum and minimum values when the user C used the body composition measuring apparatus 101 according to the present invention was 0.2 points. As is understood from the above description, it was confirmed that according to the body composition measuring apparatus of the present invention, a variation in the measurement results was suppressed significantly even if a variation in the contact position between the body and the electrodes occurred because of displacement of the standing position.

The present invention is not limited to the embodiments which have been described in detail above and can be modified into various forms as long as it has constitutions described in the claims. That is, the shape and arrangement of the electrodes may be a shape and arrangement other than those illustrated in Figs. 4 and 5. Further, in addition to a body composition measuring apparatus of a type which contacts electrodes with the bottoms of the feet of a user, the present invention can also be applied to various other types of body composition measuring apparatuses, such as a type which contacts electrodes with the palms, a type which contacts electrodes with both the palms and the bottoms of the feet, a type which contacts electrodes with body parts other than the hands and feet.

## Claims

1. A body composition measuring apparatus comprising:
at least a pair of current passing electrodes for passing a current between two body parts of a user,
at least a pair of voltage measuring electrodes for measuring a potential difference between the two body parts, and
amain unit having a surface on which these electrodes are disposed such that they are insulated from each other,
wherein
the electrodes are disposed on the surface of the main unit such that each of the current passing electrodes makes contact with a body at multiple points sandwiching a contact point between the voltage measuring electrode and the body and each of the voltage measuring electrodes makes contact with the body at multiple points sandwiching a contact point between the current passing electrode and the body.

2. The apparatus according to claim 1, wherein the current passing electrodes and the voltage measuring electrodes are disposed on the top surface of the main unit in pairs so that they make contact with the bottoms of the left and right feet of the user to pass a current between both feet and measure a potential difference between both feet.

3. The apparatus according to claim 2, wherein the current passing electrodes and the voltage measuring electrodes which make contact with the bottoms of the feet of the user in pairs each are formed in the form of a comb when viewed from above and are disposed such that their teeth engage each other.

4. The apparatus according to claim 2, wherein the current passing electrodes and the voltage measuring electrodes which make contact with the bottoms of the feet of the user in pairs each are formed in the form of small pieces when viewed from above and are disposed such that small electrode pieces for passing a current and small electrode pieces for measuring a voltage are disposed alternately.

5. The apparatus according to claim 4, wherein the small electrode pieces are round-shaped when viewed from above.

6. The apparatus according to claim 4, wherein the small electrode pieces are stick-shaped when viewed from above.
